(19) Europäisches Patentamt
European Patent Office
Office européen des brevets

(11) Publication number: 0 210 705 B1

(12) # EUROPEAN PATENT SPECIFICATION

(45) Date of publication of the patent specification: 22.03.89

(51) Int. Cl.⁴: **B01J 23/64**, B01J 23/22, C07C 45/34, C07C 47/06

(21) Application number: 86201290.3

(22) Date of filing: 22.07.86

(54) **Catalyst and process for the catalytic heterogeneous gasphase oxidation of alkenes and cycloalkenes by the Wacker route.**

(30) Priority: 27.07.85 NL 8502144

(43) Date of publication of application: 04.02.87 Bulletin 87/6

(45) Publication of the grant of the patent: 22.03.89 Bulletin 89/12

(84) Designated Contracting States: BE CH DE FR GB IT LI NL SE

(56) References cited:
DE-A- 1 417 648
FR-A- 2 073 229
US-A- 3 830 757

(73) Proprietor: **STAMICARBON B.V., Mijnweg 1, NL-6167 AC Geleen(NL)**

(72) Inventor: **Scholten, Joseph Johannes Franciscus, Sartonlaan 7, NL-6132 BC Sittard(NL)**
Inventor: **van der Steen, Petrus Johannes, Dr. G. Knuttelpark 59, NL-2552 LK Den Haag(NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a catalyst as well as a process for the catalytic heterogeneous gas-phase oxidation of an alkene or cycloalkene to the corresponding ketone or aldehyde in the presence of $H_2O$ and $O_2$, the catalyst containing a vanadium and a palladium component mounted on a carrier. Such a process is also referred to as 'heterogeneous Wacker oxidation'. Ethylene, for instance, can be oxidized to acetaldehyde, butylene-1 to methyl ethyl ketone, and cyclohexene to cyclohexanone. The reaction takes place with palladium-2+ as catalyst. For the Wacker oxidation of ethylene, the reactions taking place can be indicated as follows:

$$Pd^{2+} + CH_2 = CH_2 + H_2O \rightarrow CH_3\text{-}CHO + Pd¤ + 2H^+$$

$$Pd¤ + \text{oxidizing agent} + 2H^+ \rightarrow Pd^{2+} + \text{reduced oxidizing agent}$$

$$\text{reduced oxidizing agent} + 1/2\ O_2 \rightarrow \text{oxidizing agent} + H_2O$$

---

$$CH_2 = CH_2 + 1/2\ O_2 \rightarrow CH_3CHO$$

Such a process is known from DE-B 2 065 585, which describes the $H_2PdCl_4/V_2O_4/V_2O_5$ system on a carrier material as catalyst for the heterogeneous Wacker oxidation of olefins to aldehydes and ketones; this was later also described in J. of Catalysis 30, 109–17 (1973); as redox system, use is made of vanadium $5^+$ / vanadium $4^+$.

The disadvantage of such a catalyst, however, is its unstable behaviour. This phenomenon is reported in an article by L. Forni et al. (Ind. Engng. Chem.; Proc. Des. Dev.16 288–93 (1977): in a period of about 200 hours, the activity decreases by 50%, necessitating catalyst regeneration at about 350°C.

The carrier that in the majority of cases is used for said catalyst is α-alumina, which is a carrier with a small specific surface (up to 20 $m^2/g$), the outer layer consisting of $O^{2-}$ ions. It has been found (L. Forni, loc. cit.) that this requires the use of catalysts having a small specific surface (0.5–1.5 $m^2/g$), since at larger specific surfaces the small pores are filled up or clogged with the divanadium pentoxide.

The above-mentioned and other disadvantages of the known heterogeneous Waker oxidation are obviated by the use according to the subject invention of a catalyst, the carrier surface of which is covered with hydroxyl groups, while the vanadium is present in the form of a surface vanadate. Here and hereinafter the term 'surface vanadate' is used in the same sense as in the article by F. Roozeboom c.s. (Z. Anorg. Allg. Chem. 449, 25–40 (1979)). For the catalyst according to the invention, a chemical bond of the vanadium to the carrier is obtained in the form of a molecularly disperse layer of a vanadium oxide on the carrier. Besides the components already mentioned, the palladium component, for instance dihydropalladium tetrachloride ($H_2PdCl_4$), is present, deposited according to known techniques.

In itself, the preparation of a vanadium oxide – monolayer catalyst on a carrier with hydroxyl groups is known from the publication by F. Roozeboom c.s. (loc. cit.). Such catalysts have been described to be active for the oxidation of carbon monoxide and methanol.

Surprisingly, it has now been found that such a surface vanadate catalyst, provided with an amount of $H_2PdCl_4$, is a suitable and stable catalyst for the heterogeneous Wacker oxidation.

As carrier material use may be made of, for instance, oxides such as $Cr_2O_3$, $TiO_2$, $CeO_2$, $ZrO_2$ and γ-alumina, all of which contain hydroxyl groups on their surface. By preference, use is made of γ-alumina, which, by the chamical bond to the vanadium, yields a surface aluminium vanadate, in contrast with state-of-the-art α-alumina carriers. US-A 3 240 805 discloses that, according to the state of the art, it should be attempted to deposit the vanadium, as di-vanadium pentoxide, on the α-alumina in the form of crystalline particles having a size < 1 μm, which of course is something quite different from a surface vanadate.

From the publication by N.K. Nag et al. (Appl. Catalysis, 9, 225–33 (1984) it is known to prepare a molecularly disperse aluminium vanadate by impregnation of γ-$Al_2O_3$ (180 $m^2/g$) with an acidified stoichiometric solution of $NH_4VO_3$, the amount of vanadium that can be deposited being up to 12 wt.%. The BET surface area, calculated per carrier weight unit, then remains constant, a further indication of the formation of a monolayer, with at most one vanadium group being bound to three hydroxyl groups of the carrier according to the schedule:

1) 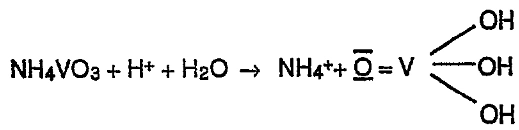

-A-

2) 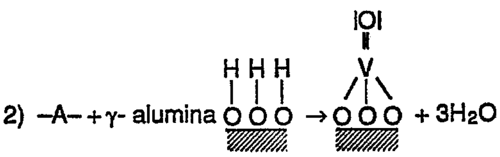

Such a surface vanadate is distinguished from $V_2O_5$ also in spectroscopic respect (N.K. Nag, loc. cit.).

The vanadium being present on the carrier surface in a monomolecular dispersion, for the Wacker synthesis now also use can be made of carrier materials having a large specific surface. By preference carrier materials with surface areas of 100–400 $m^2/g$ are applied.

Spectrometric examination (XPS/AES) of both fresh and spent catalyst, prepared using $H_2PdCl_4$, has shown the atomic ratio between chlorine and palladium to be 4, so that it must be concluded that the palladium must be present in the catalyst as $H_2PdCl_4$, and not in the form of a palladium that is chemically bound to the vanadium, as is suggested in the publication by Evnin et al. (Journal of Catalysis, 30, 109–117 (1973), loc. cit.).

The amount of vanadium that can be applied depends on the type of carrier material (number of OH groups per $m^2$) and on the specific surface of the carrier (J.B. Peri (J. Phys. Chem. 69 211 (1965)) reports that after drying at 150°C γ-alumina only has chemically bound OH groups on its surface, viz. approx. $12.5 \times 10^{18}$ OH groups per $m^2$). By preference a vanadium content of 1–30 wt.%, relative to the carrier material, is applied. The amount of palladium preferably is 0.01–3 wt.%, relative to the carrier material.

Preparation of the catalyst according to the invention preferably takes place in two steps:

a) preparation of the monolayer dispersion (the surface vanadate); this can be done by any of the techniques suitable for this, for instance the liquid method (F. Roozeboom et al., loc. cit.), in which, for instance, a solution of ammonium metavanadate ($NH_4VO_3$) is brought at a pH of 4 with $H_2SO_4$, after which this solution is passed through a column filled with carrier material until the metavanadate is no longer being bound and the solution leaves the column unchanged. Starting from gaseous $V_2O_3(OH)_4$, also a vanadium monolayer dispersion can be prepared (F. Roozeboom et al., loc. cit.).
Other methods to obtain a surface vanadate can also be used.

b) Preparation of the Pd-V carrier-catalyst: a carrier thus loaded is washed, air-dried and calcined. By dry impregnation (such that the volume of the impregnation liquid is at most equal to the pore volume), a palladium-containing solution, e.g. an $H_2PdCl_4$ solution, is deposited until the caking point (the point at which the volume of liquid added equals the pore volume of the carrier) is reached, after which the catalyst is dried.

The catalyst prepared in this way is eminently suitable for heterogeneous gas-phase oxidation by the Wacker process of alkenes or cycloalkenes, whether substituted or not.

Alkenes which can be oxidation according to the invention in particular are $C_2$–$C_{12}$ alkenes, whether or not they are substituted with substituents that do not affect the reaction, as well as $C_6$–$C_{12}$ cycloalkenes likewise whether they are substituted or note. As alkene or cycloalkene substituents, in particular groups having an electron-attracting effect may be present, such as $-C\equiv N$ group, a –halogen group, or an $-NO_2$ group. Dienes of $C_3$–$C_{12}$, such as, for instance, butadiene-1,3 and pentadiene-1,4, can also be oxidized by the subject process to the corresponding α-aldehyde, whereby a double band migration in the molecule takes place. It is preferred to use cyclohexene as cyclic alkene, but cyclopentene and cycloheptene can also be oxidized by the process according to the invention.

The temperature and pressure at which the reactions are carried out must always be chosen such that the reactants to be supplied are gaseous under those chosen conditions. The temperature, therefore, is in the 20–300°C range, while the pressure is between 0.1 and 10 MPa.

Though the invention is not tied up with any theoretical explanation, we assume that under said process conditions the pores of the catalyst carrier are wholly or partly filled via capillary condensation with, inter alia, the water present in the reaction gas, the degree of pore filling being determined by, inter alia, the process conditions and the pore size distribution. This makes it likely that the palladium is present as a homogeneous catalyst in the capillary-condensed liquid during alkene or cycloalkene

oxidation, while the fixed vanadium is responsible for the reoxidation of the palladium reduced during the oxidation. Besides the intrinsic activity for a certain alkene or cycloalkene, the catalyst activity therefore depends on the solubility of the alkene or cycloalkene in the capillary-condensed liquid (mainly water).

In a special embodiment of the invention, solubility promoters are applied to increase the said solubility of the alkene or cycloalkene to be oxidized. Examples of suitable promoters are $C_1$–$C_8$ alcohols, in particular methanol or ethanol, and further dioxane, dimethyl sulphoxide, sulpholane. Such a substance may, for instance, be dosed to the reactor in the gaseous form.

It has, surprisingly, been found that in an oxidation, according to the invention, of alkenes or cycloalkenes it is desirable for the carrier surface to be covered with vanadium to the fullest possible extent, so as to achieve maximum selectivity to the desired product. It is, therefore, preferred to aim at a degree of coverage of more than 0.25 vanadium atoms per OH surface group.

The invention will be elucidated on the basis of the following, non-restrictive examples. In the figures relating to the examples, the abscissa givens the duration of the reaction (t) in hours, and the ordinate the ethylene conversion to acetaldehyde (x) in mole%.

Example I

6 g γ-alumina of Akzo (type 000–1.5 E, S (BET) = 180 $m^2/g$) was dried at 150°C to constant weight in a 24-hour period. The material was introduced into a column and irrigated with distilled water up to a point just above the catalyst bed. After this, a 1 wt.% $NH_4VO_3$ solution (150 ml/h) of pH 4 (sulphuric acid) was passed over during 24 hours at a preferred temperature of 80°C until the ammonium metavanadate broke through.

After removal and washing of the loaded carrier, a degree of loading of approx. 8.5 wt.% surface vanadate, calculated as $V_2O_5$, per unit carrier weight, was found to have been attained.

The loaded γ-$Al_2O_3$ was washed with distilled water and air-dried at 80°C. The material was then calcined at 400°C during 1 hour and stored in the air at 150°C.

$H_2PdCl_4$ was subsequently deposited by dry impregnation with an $H_2PdCl_4$ solution containing 18 mg Pd per $cm^3$ until the caking point was reached. The caking point was reached at 0.45 $cm^3/g$, corresponding with 0.8 wt.% Pd per g catalyst. This was followed by drying at 120°C.

The Wacker oxidation of ethylene was carried out, using the catalyst obtained, in a setup with a continuous flow, the product stream being subjected to continuous gas chromatographic analysis. A microreactor with a length of 10 cm and a diameter of 0.9 cm was charged with 3 g catalyst, after which the catalyst was pre-treated in the reactor at 125°C with 116 $cm^3$ (NTP) gas per minute, the molar ratio of this gas stream being 6.95% parts water, 70.48% parts nitrogen and 22.57% parts oxygen, the total pressure being 1.2 bar and the duration 2 hours.

After this, the composition of the gas feed-stream was changed to 0.01 part $C_2H_4$, 0.23 part $H_2O$, 0.18 part $O_2$ and 0.58 part $N_2$. The reaction temperature was 140°C. The gas flow rate was set at 137 $cm^3$ (NTP) per minute, and the total pressure was 1.2 bar.

Figure 1 presents the acetaldehyde production versus time. Acetaldehyde was produced at a constant and stable rate during 18 hours, with a $C_2H_4$ conversion to $CH_3CHO$ at 19%. The material balance proved that no by-products were formed (certainly less than 5%).

Example II

The catalyst (3 g) was prepared in the same way as in Example I, but the degree of loading with surface vanadate, calculated as $V_2O_5$, was 9.5 wt.%.
The pre-treatment was as in Example I.
Reaction temperature: 125°C; pressure 1.1 bar.
Reactor feed: 130 $cm^3$ (NTP) gas per minute, 0.01 aprt $C_2H_4$, 0.18 part $H_2O$, 0.19 part $O_2$, 0.62 part $N_2$.

Figure 2 shows how the reaction proceeded in time. There is a constant $C_2H_4$ conversion to $CH_3CHO$ of 33% during 25 hours. The material balance proved that the amount of by-products formed, if any, was less than 5%.

Example III

To demonstrate that a higher ethylene concentration in the feed is also allowable, the experiment of Example II was repeated with 0.032 part $C_2H_4$, 0.14 part $H_2O$, 0.20 part $O_2$ and 0.628 part $N_2$.

The $C_2H_4$ conversion to $CH_3CHO$ now contantly was 48% during 25 hours.

Comparative experiment A

The catalyst consisted of $V_2O_5$ particles deposited on porous α-$Al_2O_3$. The catalyst preparation is described in the article by Evnin (J. Catal., 30, 109–17 (1973)); the $V_2O_5$ content was 5.5 wt.%. The reaction conditions were as follows:

reaction temperature 140°C; 3 g catalyst; feed flow rate 138 $cm^3$ (NTP) $min^{-1}$; total pressure 1.1 bar; feed composition: 0.01 part $C_2H_4$, 0.23 part $H_2O$, 0.18 part $O_2$ and 0.58 part $N_2$.

Fig. 3 shows that the conversion started at 17% $C_2H_4$ conversion to acetaldehyde, but this conversion was not stable; after 15 hours it had dropped to 2%.

Comparative experiment B

The same as Comparative experiment A, but with 6 wt.% $V_2O_5$. Conversion started at 20%, but had dropped to 6% after 4 hours (see Fig. 4).

Example IV

The heterogeneous Wacker oxidation of cyclohexene was studied using a catalyst of the following composition:
carrier material: γ-alumina of Akzo, type 000–1.5 E, with S(BET) = 253.2 $m^2/g$. The carrier was loaded with a surface vanadate corresponding with 9.5 wt.% $V_2O_5$ in the way described in Example I. By dry impregnation, subsequently an amount of $H_2PdCl_4$ corresponding with 0.8 wt.% Pd was deposited. The catalyst was air-dried at 80°C during one hour.

An amount of 3 g catalyst was pre-treated in the reactor at 125°C in a gas stream, fed at 116 $cm^3$ (NTP) per minute, composed of 6.95% water vapour, 70.48% nitrogen and 22.75% oxygen. The total pressure was 1.1 bar. The pre-treatment lasted two hours.

Afterwards, the following feed was passed over:
0.8% cyclohexene, 19.2% oxygen, 10.4% water vapour and 70% nitrogen. The glas flow rate was 127 $cm^3$ (NTP) per minute. After two hours the initial reactor temperature of 110°C was lowered to 75°C.

The cyclohexene conversion after this amounted to 3.6%, and the reaction product consisted of 70% cyclohexanone, 24% cyclohexenone and 5% phenol.

After 40 hours' experimenting, no appreciable change in conversion or in selectivities was observed.

**Claims**

1. Catalyst, suitable for the catalytic heterogeneous gas phase oxidation of an alkene or cycloalkene to the corresponding ketone or aldehyde in the presence of $H_2O$ and $O_2$, containing a vanadium and a palladium component, mounted on a carrier, characterized in that the surface of the carrier is covered with hydroxyl groups and the vanadium is present in the form of a surface vanadate.

2. Catalyst according to claim 1, characterized in that the carrier consists of γ-alumina.

3. Catalyst according to either of claims 1–2, characterized in that the carrier has a surface area of 100–400 $m^2/g$.

4. Catalyst according to claim 1, characterized in that the vanadium content of the catalyst is 2–30 wt.%., relative to the carrier material.

5. Catalyst according to claim 4, characterized in that the degree of coverage with vanadium of the catalyst is higher than 0.25 vanadium atom per surface hydroxyl group.

6. Process for the preparation of a catalyst according to claim 1, characterized in that a carrier with surface hydroxyl groups is started from, a monolayer dispersion of vanadium is deposited, which is followed by dry impregnation with a palladium-containing solution until the caking point is reached.

7. Process for the heterogeneous Wacker oxidation of an alkene or a cycloalkene, characterized in that use is made of a catalyst according to claim 1.

8. Process according to claim 7, characterized in that the oxidation is carried out at a temperature of 20–200°C.

9. Process according to claim 7, characterized in that a pressure of 0.01–10 MPa is applied.

10. Process according to claim 7, characterized in that use is also made of a solubility promoter for the alkene or cycloalkene in the capillary condensing liquid.

11. Process according to claim 10, characterized in that methanol or ethanol is used as solubility promoter.

**Patentansprüche**

1. Katalysator, der für die katalytische heterogene Gasphasenoxidation eines Alkens oder Cycloalkens zum entsprechenden Keton oder Aldehyd in Anwesenheit von $H_20$ und $0_2$ geeignet ist und der eine Vanadium- und eine Palladiumkomponente enthält, die auf einem Träger angeordnet sind, dadurch gekennzeichnet, daß die oberfläche des Trägers mit Hydroxylgruppen bedeckt ist und das Vanadium in Form eines Oberflächenvanadats vorhanden ist.

2. Katalysator nach Anspruch 1, dadurch gekennzeichnet, daß der Träger aus γ-Aluminiumoxid besteht.

3. Katalysator nach einem der Ansprüche 1 bis 2, dadurch gekennzeichnet, daß der Träger eine Oberfläche von 100 bis 400 $m^2/g$ aufweist.

4. Katalysator nach Anspruch 1, dadurch gekennzeichnet, daß der Vanadiumgehalt des Katalysators 2 bis 30 Gew.%, bezogen auf das Trägermaterial, beträgt.

5. Katalysator nach Anspruch 4, dadurch gekennzeichnet, daß der Bedeckungsgrad des Katalysators mit Vanadium höher als 0,25 Vanadiumatom pro Oberflächenhydroxylgruppe ist.

6. Verfahren zur herstellung eines Katalysators nach Anspruch 1, dadurch gekennzeichnet, daß von einem Träger mit Oberflächenhydroxylgruppen ausgegangen wird und eine Monoschichtdispersion von Vanadium abgesetzt wird, worauf mit einer palladiumhaltigen Lösung trocken imprägniert wird, bis der caking point erreicht ist.

7. Verfahren zur heterogenen Wacker-Oxidation eines Alkens oder eines Cycloalkens, dadurch gekennzeichnet, daß ein Katalysator nach Anspruch 1 verwendet wird.

8. Verfahren nach Anspruch 7, dadurch gekennzeichnet, daß die Oxidation bei einer Temperatur von 20 bis 200°C durchgeführt wird.

9. Verfahren nach Anspruch 7, dadurch gekennzeichnet, daß ein Druck von 0,01 bis 10 MPa angewandt wird.

10. Verfahren nach Anspruch 7, dadurch gekennzeichnet, daß auch ein Löslichkeitspromotor für das Alken oder Cycloalken in der kapillarkondensierenden Flüssigkeit verwendet wird.

11. Verfahren nach Anspruch 10, dadurch gekennzeichnet, daß Methanol oder Äthanol als Löslichkeitspromotor verwendet wird.

## Revendications

1. Catalyseur approprié pour l'oxydation catalytique hétérogène en phase gazeuse d'un alcène ou cycloalcène en cétone ou aldéhyde correspondant en présence de $H_20$ et $0_2$, contenant un composant vanadium et un composant palladium, montés sur un support, caractérisé en ce que la surface du support est recouverte de groupes hydroxyle et que le vanadium est présent sous forme d'un vanadate à la surface.

2. Catalyseur selon la revendication 1, caractérisé en ce que le support est la gamma-alumine.

3. Catalyseur selon la revendication 1 ou 2, caractérisé en ce que la surface de contact du support est de 100 à 400 $m^2/g$.

4. Catalyseur selon la revendication 1, caractérisé en ce que la teneur en vanadium du catalyseur est de 2 à 30% en poids par rapport au support.

5. Catalyseur selon la revendication 4, caractérisé en ce que le degré de recouvrement avec le vanadium du catalyseur est supérieur à 0,25 atome de vanadium par groupe hydroxyle à la surface.

6. Procédé de préparation d'un catalyseur selon la revendication 1, caractérisé en ce qu'on part d'un support portant des groupes hydroxyle en surface, on dépose une dispersion en monocouche de vanadium, qu'on fait suivre d'une imprégnation à sec avec une solution de palladium jusqu'à atteindre le "caking point".

7. Procédé d'oxydation hétérogène Wacker d'un alcène ou cycloalcène, caractérisé en ce qu'on utilise un catalyseur selon la revendication 1.

8. Procédé selon la revendication 7, caractérisé en ce qu'on effectue l'oxydation à une température de 20 à 200°C.

9. Procédé selon la revendication 7, caractérisé en ce qu'on applique une pression de 0,01 à 10 MPa.

10. Procédé selon la revendication 7, caractérisé en ce qu'on utilise également un agent promoteur de solubilité pour l'alcène ou le cycloalcène dans le liquide de condensation capillaire.

11. Procédé selon la revendication 10, caractérisé en ce qu'on utilise du méthanol ou de l'éthanol en qualité d'agent promoteur de solubilité.

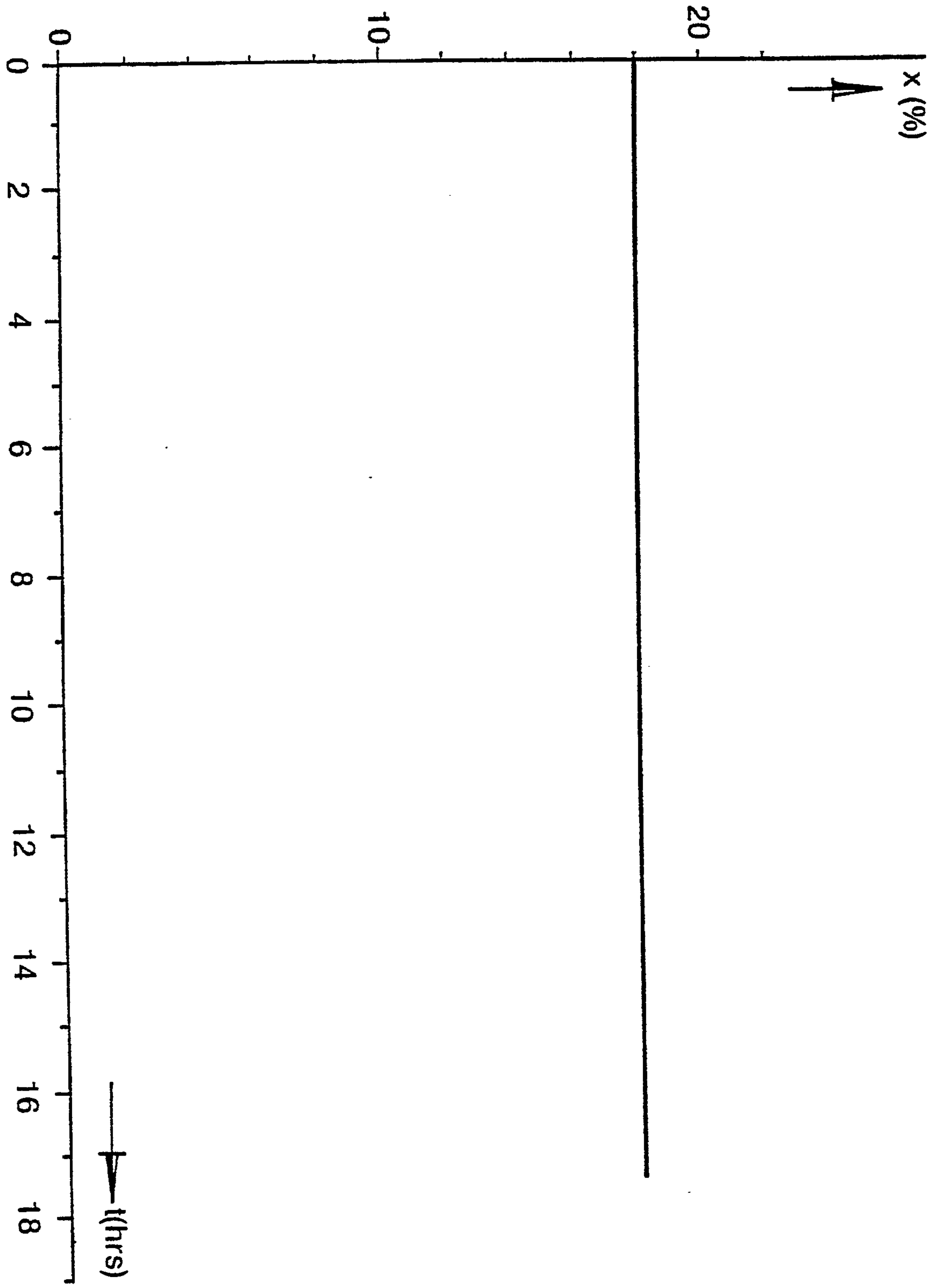
x (%)
0
10
20
t(hrs)
0
2
4
6
8
10
12
14
16
18

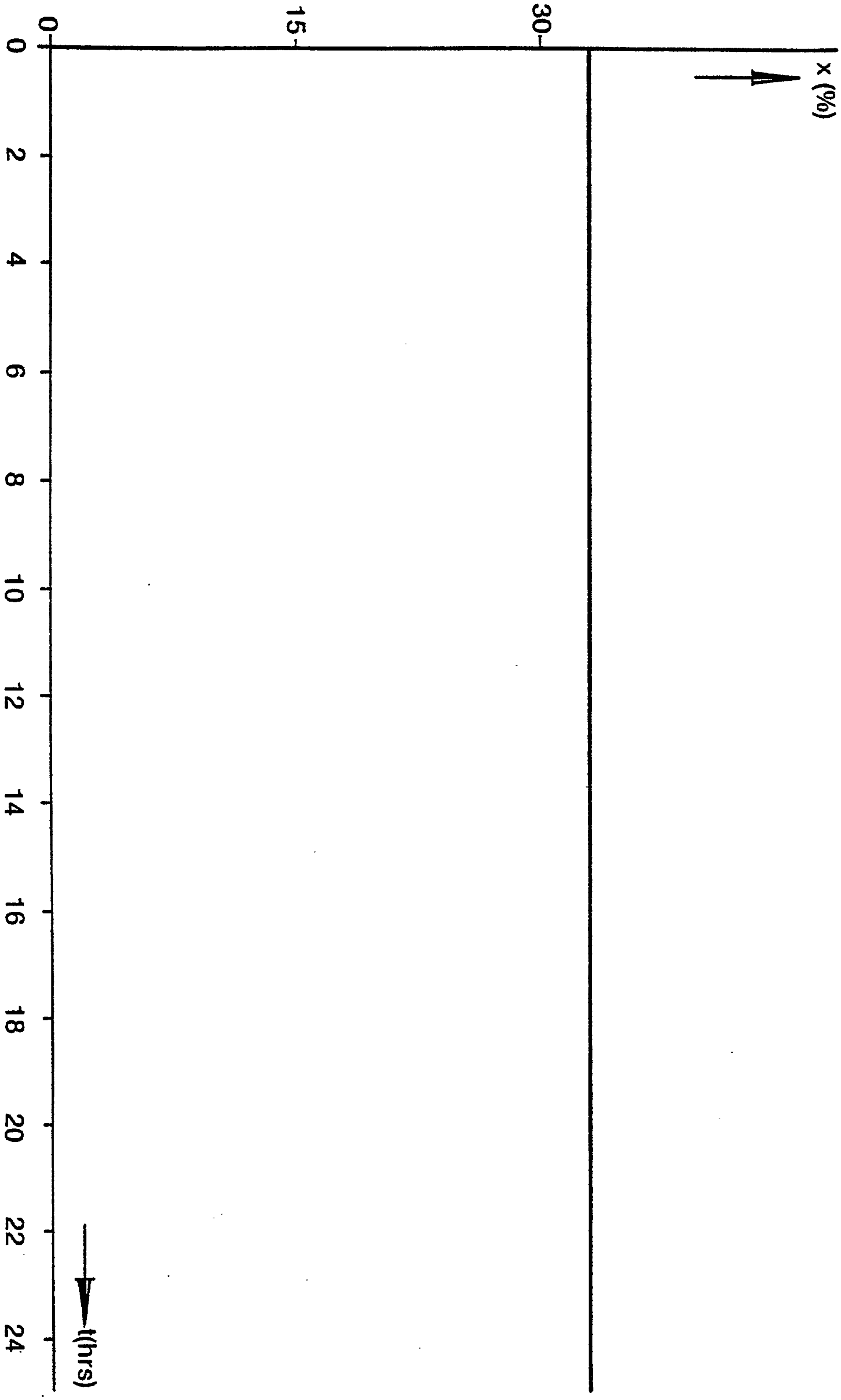
0
15
30
x (%)
0
2
4
6
8
10
12
14
16
18
20
22
24
t(hrs)

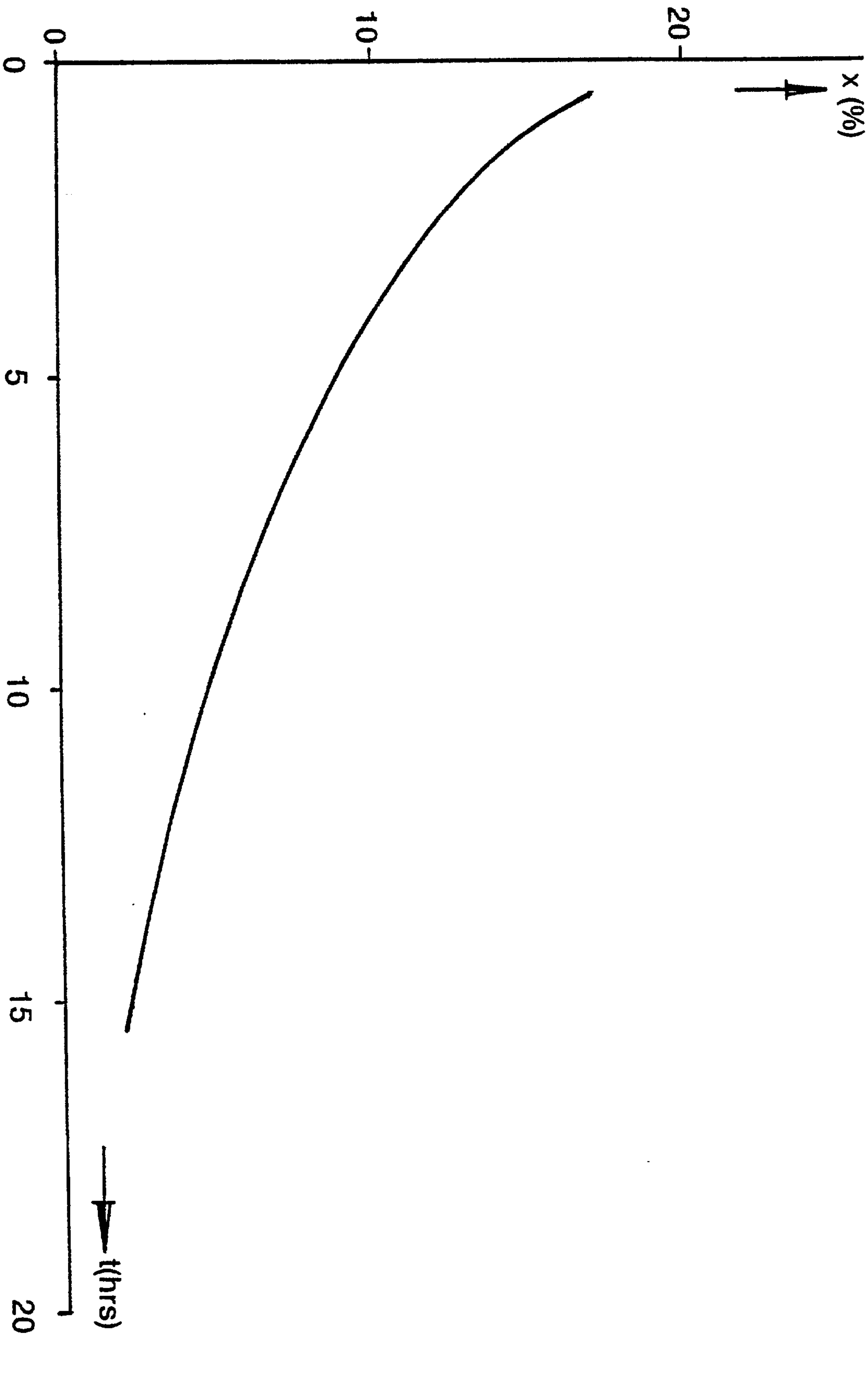
x (%)
0
10
20
t(hrs)
0
5
10
15
20

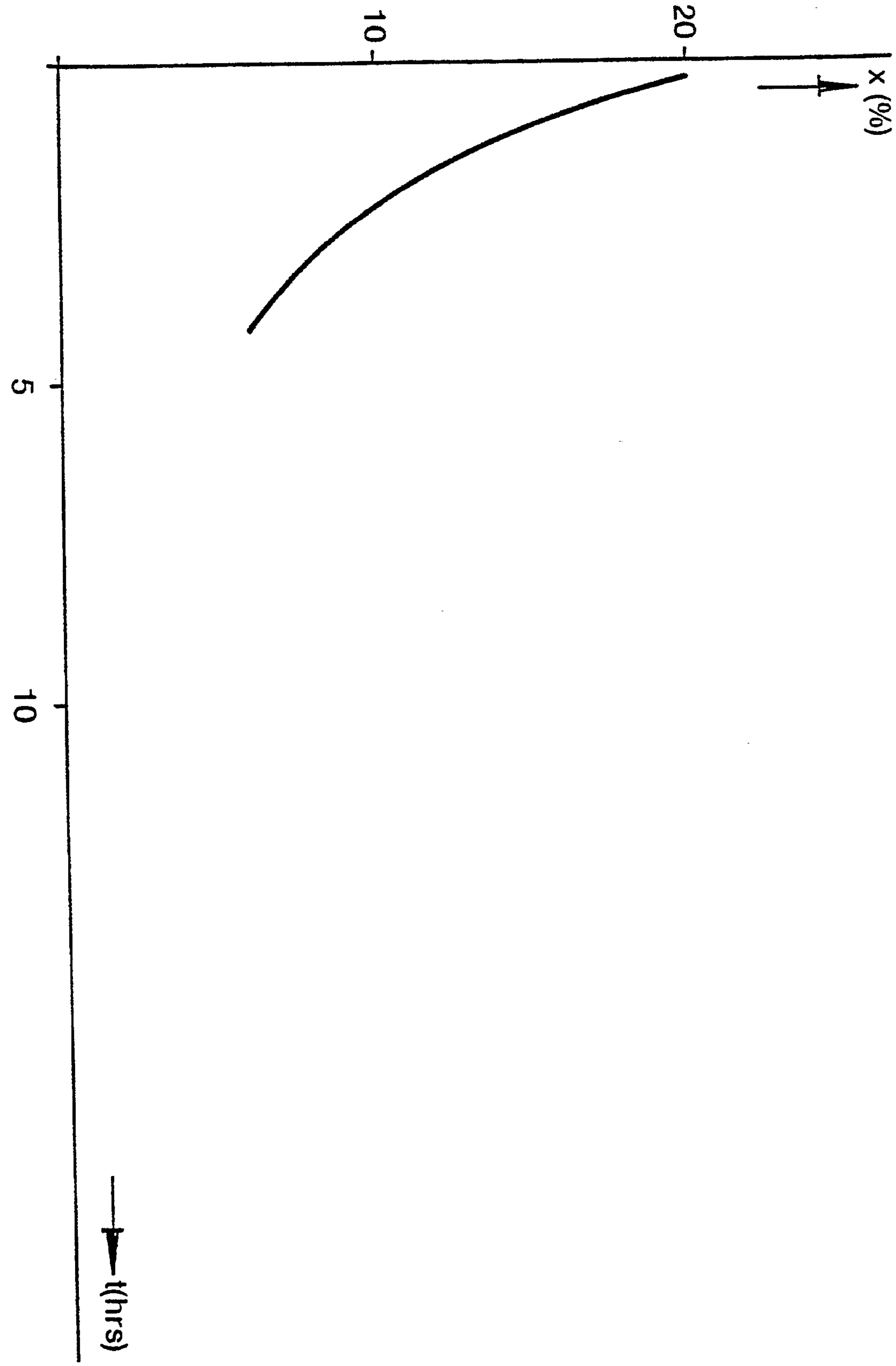
x (%)
20
10
5
10
t(hrs)